# EUROPEAN PATENT APPLICATION

(11) **EP 4 484 554 A1**
(43) Date of publication of application: **01.01.2025**
(21) Application number: 23760434.3
(22) Date of filing: 27.02.2023
(51) Int. Cl.: C12N 5/0775, C12N 5/00

(54) **METHOD FOR CULTURING STEM CELLS USING MICROCARRIER**

(30) Priority: 25.02.2022 KR 20220025325; 25.02.2022 KR 20220025326
(71) Applicant: Kangstem Biotech Co., Ltd., Seoul 06179 (KR)
(72) Inventor: KIM, Mijin, Seoul 08590 (KR); LEE, Jusik, Siheung-si, Gyeonggi-do 15027 (KR); AHN, Jongchan, Bucheon-si, Gyeonggi-do 14597 (KR); LEE, Seunghee, Seoul 08797 (KR); KANG, Kyungsun, Seoul 06338 (KR)
(74) Representative: Regimbeau
(86) International application number: PCT/KR2023/002743
(87) International publication number: WO 2023/163560

(57) **Abstract**

The present invention relates to a method for culturing stem cells using microcarriers.

## Description

### [Technical Field]

The present invention relates to a method for culturing stem cells using microcarriers.

### [Background Art]

Stem cells are undifferentiated cells at stages prior to differentiation that are obtainable from embryonic, fetal, and adult tissues and have characteristics of self-renewal capacity, differentiation potency, immortality, *etc.* Stem cells may be classified into pluripotent, multipotent, and unipotent stem cells according to their differentiation potency. Since adult stem cells with multipotency are relatively free from the risk of cancerization, immunorejection, and ethical concerns compared to embryonic stem cells with pluripotency and induced pluripotent stem cells (IPSCs), extensive research thereon and development thereof as therapeutic agents have been in progress. Particularly, in the case of umbilical cord blood-derived mesenchymal stem cells (UCB-MSCs), which are mesenchymal stem cells derived from the blood of newborn babies that circulates through a cardiovascular system of a fetus during pregnancy and is available from a placenta and an umbilical cord after childbirth, after research reports showing that UCB-MSCs have superior proliferation ability to that of stem cells derived from other tissues were published, UCB-MSCs have drawn considerable attention in various fields of study such as the medical field. Particularly, as stem cells separated from discarded tissues, UCB-MSCs have a high value in terms of low donor's burden and recycling of medical waste.

### [Prior Art Document]

(Patent Document 1) Korean Publication Application KR 10-2023-0017786 A

### [Disclosure]

### [Technical Problem]

Accordingly, a method for efficiently culturing stem cells is required.

### [Technical Solution]

An object of the present invention is to provide a method for culturing stem cells using microcarriers.

### [Advantageous Effects]

The culture method of the present invention can maintain stem cells in a small size, and delay or suppress senescence.

### [Brief Description of Drawings]

FIG. 1 shows the results of comparing the size of stem cells according to plate-adherent culture and microcarrier culture.
FIG. 2 shows the results of comparing the level of stem cell senescence according to plate-adherent culture and microcarrier culture.

### [Detailed Description of Preferred Embodiments]

The present invention will be described in detail as follows. Meanwhile, each description and embodiment disclosed herein can be applied to other descriptions and embodiments, respectively. That is, all combinations of various elements disclosed herein fall within the scope of the present invention. Further, the scope of the present invention is not limited by the specific description described below.

Additionally, those of ordinary skill in the art may be able to recognize or confirm, using only conventional experimentation, many equivalents to the particular aspects of the disclosure described herein. Furthermore, it is also intended that these equivalents be included in the present invention.

Additionally, a number of papers and patent documents have been cited throughout the present specification. The content of the cited papers and patent documents is incorporated herein by reference in their entirety and the level of technical field to which the present invention belongs and the contents of the present invention will be described more clearly.

One aspect of the present invention provides a method for enhancing the efficacy of stem cells, including a step of culturing stem cells while being adhered to microcarriers.

In one embodiment, the stem cells are cultured while being adhered to microcarriers floating in a cell culture solution, and the microcarriers are polygalacturonic acid (PGA) crosslinked with each other, and the method has one or more characteristics selected from (i) a decrease or maintenance of the size of cells; and (ii) a delay in senescence, compared to a plate culture.

It has been reported that when stem cells are arbitrarily separated based on a certain size, the efficacy of stem cells is increased, i.e., the proliferation ability of small cell groups is relatively high, the expression of senescence-related markers is low, *etc.* In another study, it was confirmed that stem cells with a diameter below a certain size showed a relatively excellent whitening effect, and in still another study, it has been reported that stem cells with high mobility have high differentiation potency and that these cells are relatively small in size. Accordingly, maintaining the cell size relatively small during stem cell culture can lead to enhanced efficacy of stem cells.

In addition, it has been reported that lysosomal proteins, reactive oxygen species (ROS), β-galactosidase activity, *etc.* increase in cells that are aged through stem cell culture. Therefore, in order to measure the level of senescence, any one or more of lysosomal proteins, reactive oxygen species (ROS), and β-galactosidase activity can be measured. For example, the level of senescence of stem cells can be determined by measuring β-galactosidase activity.

The culture method of the present invention according to any one of the specific embodiments described above is characterized in that (i) the size of the stem cells may be constantly maintained, and/or (ii) the senescence of the stem cells may be suppressed or delayed, as compared to when the stem cells are cultured on a plate.

The culture method of the present invention according to any one of the specific embodiments described above is characterized in that the size of the stem cells may be constantly maintained or decreased, and the senescence of the stem cells may be suppressed or delayed, as compared to when the stem cells are cultured on a plate.

In any one of the specific embodiments described above, the culture method of the present invention includes adhering stem cells to microcarriers and culturing the stem cells in a state where the stem cells are adhered to the microcarriers floating in KSB-3 Complete Medium@ Kit medium.

As used herein, "stem cell" refers to a cell capable of differentiating into various tissues, i.e., an undifferentiated cell. The stem cell may be derived from humans or animals, or may be derived from the umbilical cord, umbilical cord blood, bone marrow, fat, muscle, nerve, skin, amnion, or placenta.

In one embodiment, the stem cell of the present invention may be a mesenchymal stem cell.

In any one of the specific embodiments described above, the umbilical cord blood-derived mesenchymal stem cell may be an umbilical cord blood-derived pluripotent stem cell that exhibits positive immunological characteristics for ZNF281, a transcriptional regulatory factor, and may show the following characteristics.

In any one of the specific embodiments described above, the umbilical cord blood-derived mesenchymal stem cell may show any one or more characteristics from the following:
(a) shows positive immunological characteristics for c-myc, a transcriptional regulatory factor;
(b) adheres to the extracellular matrix-coated surface and proliferates while forming cell colonies in the form of a line or sphere within 5 to 30 days after adherence;
(c) shows a cumulative population doubling level (CPDL) of 30 to 45;
(d) shows negative immunological characteristics for CD14, CD31, CD34, CD45, and HLA-DR;
(e) is capable of differentiating into mesoderm, endoderm, and ectoderm cells; and
(f) secretes at least one cytokine or chemokine selected from the group consisting of TIMP-2, TGF-β, RANTES CINC-3, EOTAXIN, GM-CSF, IFN-γ, IL-1b, IL-3, IL-6, IL-8, IL-10, IL12p40, IL13, IL-16, IP-10, Leptin, MCP-2, MIG, MIP-3a, b-NGFm, sTNFRI, and PFGF-bb.

In any one of the specific embodiments described above, the umbilical cord blood-derived mesenchymal stem cell may be a cell obtained by separation as disclosed in Korean Patent No. 10-0950195. With respect to the umbilical cord blood-derived mesenchymal stem cells of the present invention, the contents disclosed in Korean Patent Nos. 10-0950195 and 10-1627907 are incorporated by reference.

In any one of the specific embodiments described above, the stem cell culture of the present invention may be a subculture.

As used herein, "subculture" is one of the cell proliferation methods, and is a culture method of transferring cells from a previous culture to a fresh growth medium, for example, every 1 to 7 days. Subculture is a culture method that preserves cell lines and maintains cell generations, and this action is called subculturing or passsaging. Through subculturing, accumulated toxic metabolites may be removed and depleted nutrients may be supplied, thereby inhibiting cell death and promoting growth and proliferation.

In any one of the specific embodiments described above, subculturing may be performed for two or more passages. In one specific embodiment, subculturing may be performed for 3, 4, or 5 passages, or more.

In any one of the specific embodiments described above, the culture method of the present invention may include culturing by seeding stem cells at 2,000 cells/cm² to 4,000 cells/cm². In one specific embodiment, the culture method of the present invention may include a step of seeding stem cells at about 3,000 cells/cm².

In any one of the specific embodiments described above, the culture method of the present invention may use microcarriers at a dose of 0.5 mg/ml to 2.0 mg/ml relative to the volume of medium. In one specific embodiment, the culture method of the present invention may use microcarriers at a dose of 0.5 mg/ml to 1.5 mg/ml relative to the volume of medium. In one specific embodiment, the culture method of the present invention may use microcarriers at a dose of about 1.0 mg/ml relative to the volume of medium.

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

In any one of the specific embodiments described above, the culture method of the present invention may be performed in a bioreactor. The bioreactor may include a configuration for stirring the medium.

In any one of the specific embodiments described above, the culture method of the present invention may use the KSB-3 Complete Medium@ Kit medium.

### [Mode for Carrying Out the Invention]

Hereinafter, the present invention will be described in detail by way of Examples and Experimental Examples. However, these Examples and Experimental Examples are merely preferred Examples given for illustrative purposes, and thus, the scope of the present invention is not intended to be limited to or by these Examples and Experimental Examples.

### Example 1: Comparative Analysis of the Size of Umbilical Cord Blood-derived Mesenchymal Stem Cells according to Culture Medium Conditions

In order to compare and analyze the cell size of umbilical cord blood-derived mesenchymal stem cells under a plate culture and a microcarrier-adherent culture condition, the umbilical cord blood-derived mesenchymal stem cells were seeded at 3,000 cells/ cm² in a 150 mm plate and cultured in a 5% CO₂ incubator at 37°C from P3 to P6. As disclosed in Korean Patent No. 10-0950195, the umbilical cord blood-derived mesenchymal stem cells that showed positive immunological characteristics for ZNF281, a transcriptional regulatory factor, were isolated and used.

Specifically, the preparation and culture process of the umbilical cord blood-derived mesenchymal stem cells are as follows. The frozen cells were thawed, mixed thoroughly by pipetting, suspended in KSB-3 Complete Medium@ Kit, and centrifuged at 500 g for 5 minutes at room temperature. Thereafter, the supernatant was removed so that the cell pellet remained intact, and the cells were resuspended in KSB-3 Complete Medium@ Kit to measure the cell number and viability.

The culture process on the plate is as follows. After adding 15 mL of KSB-3 Complete Medium@ Kit to a 150 mm plate, the umbilical cord blood-derived mesenchymal stem cells were added to the plate at a density of 3,000 cells/cm², and cultured in a 5% CO₂ incubator at 37°C for a total of 5 days.

The culture process in the bioreactor is as follows. Microcarriers (dissolvable microcarriers (Corning^{®} Dissolvable Microcarriers)) were weighed at 0.4 g and sterile distilled water was added thereto for hydration at 4°C. The hydrated microcarriers were placed in a bioreactor vessel (PBS Biotech Inc., 0.5L bioreactor) with a volume of 400 ml using KSB-3 complete medium and reacted at 20 RPM for 30 minutes. Umbilical cord blood-derived mesenchymal stem cells were added to the bioreactor vessel to a density of 3,000 cells/cm² and cultured in a 5% CO₂ incubator at 37°C from P3 to P6 with 15 RPM for a total of 5 days.

At each passage time, the plate-cultured cells were treated with a TrypLE reagent to obtain single cells, and the bioreactor-cultured cells adhered to microcarriers were treated with a TrypLE reagent containing pectinase to dissolve the microcarriers and then single cells were obtained. The obtained single cells were compared and analyzed for cell size using NC200.

As a result, the diameters of the cells in each culture condition in P4 were similar, which were found to be 14.15 µm and 14.50 µm under the plate culture condition and microcarrier culture condition, respectively. However, it was confirmed that in P5, the diameters of the cells were found to be 16.20 µm and 14.50 µm, respectively, showing a difference of about 1.7 µm, and in P6, the diameters of the cells were found to be 16.40 µm and 14.15 µm, respectively, showing a difference of about 2.25 µm.

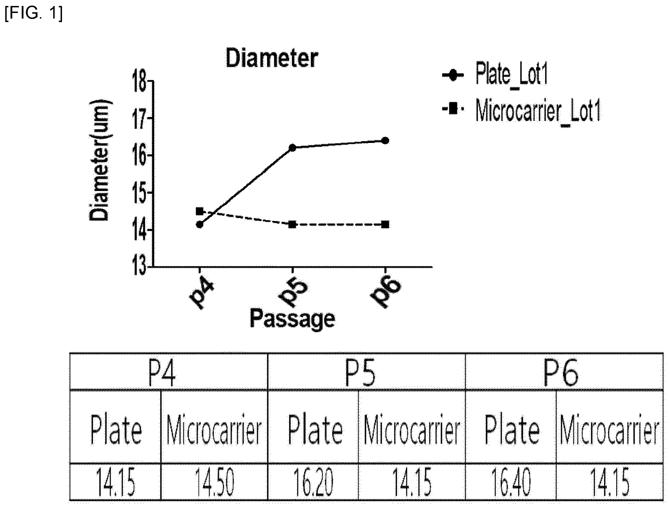

Therefore, it was confirmed that the size of the umbilical cord blood-derived mesenchymal stem cells under the plate culture increased as the passages were continued under the same conditions, whereas the size of the umbilical cord blood-derived mesenchymal stem cells under the microcarrier-adherent culture did not increase even when the passages were continued under the same conditions (FIG. 1).

### Example 2: Comparative Analysis of Senescence of Umbilical Cord Blood-derived Mesenchymal Stem Cells according to Culture Medium Conditions

In order to compare and analyze the degree of senescence of umbilical cord blood-derived mesenchymal stem cells under a plate culture and a microcarrier-adherent culture condition, the degree of senescence of the umbilical cord blood-derived mesenchymal stem cells under each culture condition was compared and analyzed through β-galactosidase staining.

The cells in each culture condition of P4, P5 and P6 obtained in Example 1 above were seeded equally in a 12-well plate at 1×10⁵ cells/mL and cultured in a 5% CO₂ incubator at 37°C for one day. Thereafter, the cells were washed once with PBS and fixed with 4% paraformaldehyde for 10 minutes. After fixation, the cells were washed twice with PBS and treated with 0.5 mL of β-galactosidase staining reagent per well. After staining for 48 hours at 37°C, the cells were examined under a microscope.

As a result of β-galactosidase staining, the SA-β Gal Positive (%) values in P4 were 15.07% and 19.28% under the plate culture condition and microcarrier culture condition, respectively, and the values in P5 were 32.49% and 28.83%, respectively, showing that the value under the microcarrier culture condition was about 4% lower, and in P6, the values were 37.95% and 27.79% respectively, showing that the value under the microcarrier culture condition was about 10% lower.

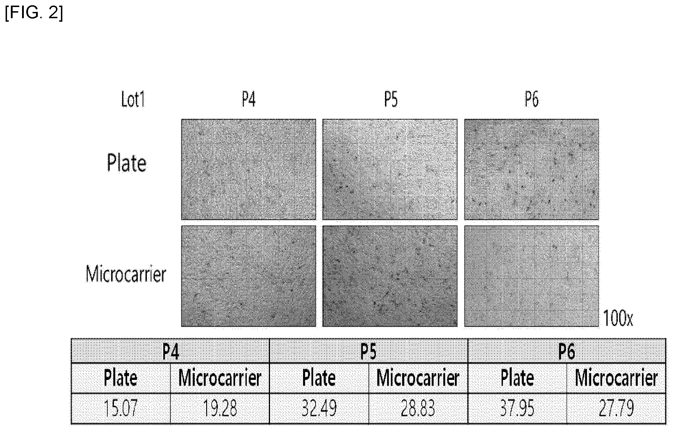

Therefore, it was confirmed that the senescence of the umbilical cord blood-derived mesenchymal stem cells was delayed in the repeated microcarrier subculture conditions, compared to the repeated plate subculture conditions (FIG. 2).

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A method for enhancing the efficacy of stem cells, comprising a step of culturing stem cells while being adhered to microcarriers,
wherein the stem cells are cultured while being adhered to microcarriers floating in a cell culture solution,
the microcarriers are polygalacturonic acid (PGA) crosslinked with each other, and
the method has one or more characteristics selected from (i) a decrease or maintenance of the size of cells; and (ii) a delay in senescence, compared to plate culture.

2. The method of claim 1, wherein the culture is performed via subculture.

3. The method of claim 1, wherein the subculture is performed for two or more passages.

4. The method of claim 1, wherein the stem cell is a mesenchymal stem cell derived from umbilical cord blood.

5. The method of claim 1, wherein the enhancement of the efficacy of stem cells is **characterized by** a decrease or maintenance of the size of stem cells and a delay in stem cell senescence.

6. The method of claim 1, wherein the stem cell is an umbilical cord blood-derived pluripotent stem cell that exhibits positive immunological characteristics for ZNF281, a transcriptional regulatory factor, and has the following characteristics:
(a) shows positive immunological characteristics for c-myc, a transcriptional regulatory factor;
(b) adheres to the extracellular matrix-coated surface and proliferates while forming cell colonies in the form of a line or sphere within 5 to 30 days after adherence;
(c) shows a cumulative population doubling level (CPDL) of 30 to 45;
(d) shows negative immunological characteristics for CD14, CD31, CD34, CD45, and HLA-DR;
(e) is capable of differentiating into mesoderm, endoderm, and ectoderm cells; and
(f) secretes at least one cytokine or chemokine selected from the group consisting of TIMP-2, TGF-β, RANTES CINC-3, EOTAXIN, GM-CSF, IFN-γ, IL-1b, IL-3, IL-6, IL-8, IL-10, IL12p40, IL13, IL-16, IP-10, Leptin, MCP-2, MIG, MIP-3a, b-NGFm, sTNFRI, and PFGF-bb.

7. The method of claim 1, wherein the method comprises culturing by seeding stem cells at 2,000 cells/cm² to 4,000 cells/cm².

8. The method of claim 1, wherein the method comprises using the microcarriers at a dose of 0.5 mg/ml to 2.0 mg/ml relative to the volume of medium.

9. The method of claim 1, wherein the method comprises culturing stem cells in a state where the stem cells are adhered to the microcarriers floating in the KSB-3 Complete Medium@ Kit medium.
